# EUROPEAN PATENT SPECIFICATION

(11) **EP 3 788 364 B1**
(45) Date of publication and mention of the grant of the patent: **11.01.2023**
(21) Application number: 18740276.3
(22) Date of filing: 04.05.2018
(51) Int. Cl.: G01N 33/18, G01N 21/3577

(54) **MEASUREMENT OF HYDROCARBON CONTAMINATION IN WATER**
MESSUNG DER KOHLENWASSERSTOFFKONTAMINATION IN WASSER
MESURE DE CONTAMINATION PAR HYDROCARBURES DANS L'EAU

(43) Date of publication of application: 10.03.2021
(73) Proprietor: ABB Schweiz AG, 5400 Baden (CH)
(72) Inventor: BUIJS, Henry L., Quebec City, G1T 1C6 (CA)
(74) Representative: Maiwald GmbH
(86) International application number: PCT/IB2018/000577
(87) International publication number: WO 2019/211640

(56) References cited:
- EP-A1- 0 176 531
- WO-A1-95/06873
- GB-A- 1 543 320
- RU-C1- 2 325 631
- US-A1- 2017 370 896

## Description

### TECHNICAL FIELD

The present application generally relates to hydrocarbon contamination and more particularly, to an apparatus and method for measuring hydrocarbon contamination in water.

### BACKGROUND

Methods and apparatuses for measuring hydrocarbon contamination in water remain an area of interest. Some existing apparatuses have various shortcomings, drawbacks and disadvantages relative to certain applications. For example, with some methods and apparatuses, a current method for measuring hydrocarbon contamination in water employs solvent or membrane extraction of the hydrocarbon from a known quantity of water, followed by determination of the hydrocarbon quantity by infrared analysis of the extracted hydrocarbon using an infrared transparent solvent, such as chlorofluorocarbon, which is time consuming.
Accordingly, there remains a need for further contributions in this area of technology.

WO 95/06873 refers to a method for determining the concentration of a component present in a fluid stream in dispersed form. The method comprises the steps of a) emitting a light beam in a predetermined range of wavelengths to a fluid sample to be analyzed, said sample flowing through a measurement cell; b) selecting a number of different wavelengths in said predetermined range; c) measuring a number of light intensities while sample fluid is flowing through the measurement cell and deriving therefrom a number of measurement sets which each consist of a number of measured light intensities at said different wavelengths; d) measuring a number of light intensities, while reference fluid is flowing through the measurement cell and deriving therefrom a number of reference fluid measurement sets which each consist of a number of measured reference light intensities at said different wavelengths; and e) deriving from the data, thus obtained, information on the concentration of dispersed components in the fluid stream.

US 2017/0370896 A1 refers to a phase fraction determination system that includes a pipe or tube through which a multiphase fluid flows, the multiphase fluid being capable of having any of three or four phases that include a gas phase, an oil phase, a water phase, and a solid phase. The gas phase and the oil phase are hydrocarbon bearing. A window is formed in the pipe. The window is optically transparent to the wavelengths of collimated light emitted by collimated light sources. The collimated light sources are laser light sources employing laser diodes and emitting laser light in the near-infrared wavelength spectrum. Each laser emits light in a different narrow wavelength spectrum.

RU 2 325 631 C1 relates to a method that implies determining an oil and water concentration based on a set of equations that involves three different wavelengths.

### SUMMARY

The present invention provides a unique method for performing infrared analysis for measuring hydrocarbon contamination in water, as defined in claim 1. The invention further provides a unique apparatus for performing infrared analysis for measuring hydrocarbon contamination in water, as defined in claim 9. Preferred embodiments are defined by the dependent claims. Further embodiments, forms, features, aspects, benefits, and advantages of the present application shall become apparent from the description and figures provided herewith.

### BRIEF DESCRIPTION OF THE FIGURES

The description herein makes reference to the accompanying drawings wherein like reference numerals refer to like parts throughout the several views, and wherein:
FIG. 1 schematically illustrates some aspects of a non-limiting example of an apparatus for measuring hydrocarbon contamination in water in accordance with an embodiment of the present invention.
FIG. 2 is a plot illustrating some aspects of a non-limiting example of water absorbance in the near infrared spectrum based on 10 µm and 100 µm sample path lengths.
FIG. 3 is a plot illustrating some aspects of a non-limiting example of water absorption and hydrocarbon absorption in the near infrared spectrum highlighting a region of low water absorption and high hydrocarbon absorption.
FIG. 4 illustrates some aspects of a non-limiting example of a transmittance spectrum of a 5 mm path length water sample in the near infrared, providing about 5% transmittance in the approximately 5700 cm⁻¹ to 6300 cm⁻¹ region.
FIG. 5 illustrates some aspects of a non-limiting example of a transmittance region at approximately 5700 cm⁻¹ to 6300 cm⁻¹ defined by water absorption on both the high and low frequency side of the region.
FIG. 6 illustrates some aspects of a non-limiting example of absorbance spectra for four (4) traces of 20 ppm isopropanol in water and three (3) traces of absorbance for pure water.

### DETAILED DESCRIPTION OF THE ILLUSTRATIVE EMBODIMENTS

For the purposes of promoting an understanding of the principles of the invention, reference will now be made to the embodiments illustrated in the drawings and specific language will be used to describe the same. It will nevertheless be understood that no limitation of the scope of the invention is thereby intended.

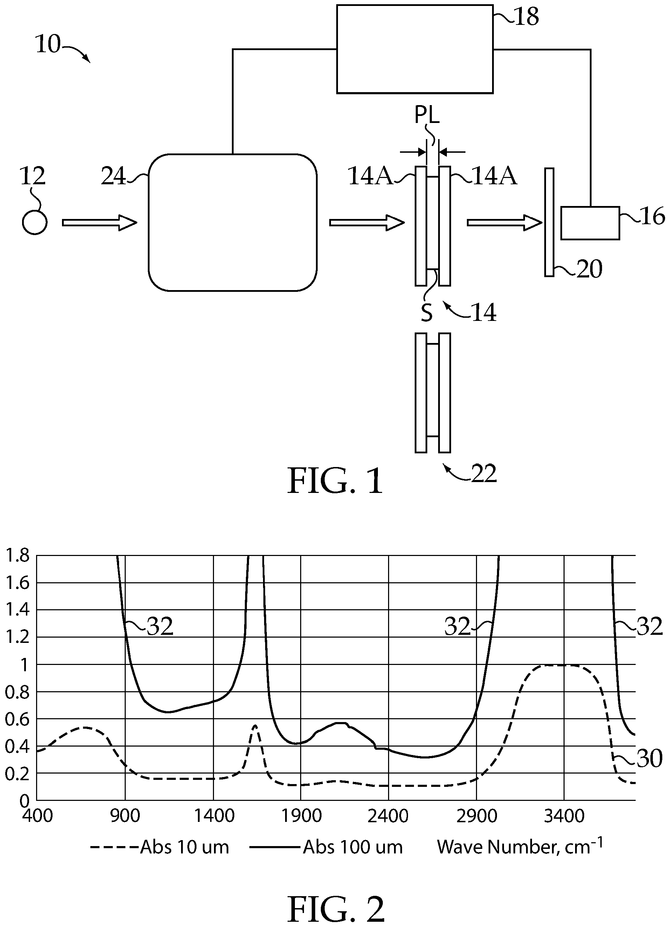

Referring to FIG. 1, some aspects of a non-limiting example of an apparatus 10 for measuring hydrocarbon contamination in water in accordance with an embodiment of the present invention are schematically illustrated. Apparatus 10 includes a light source 12, a sample cell 14; a detector 16 and a controller 18. Some embodiments may include a filter 20, e.g., disposed in the optical path between the sample cell and detector 16. Other embodiments may not include a filter or may include other filters in addition to or in place of filter 20. In some embodiments light source 12 may include one or more filters, or one or more filters may be disposed between light source 12 and the sample cell, so that the sample cell receives light at only one or more desired wavelengths or wavelength bands. Some embodiments may include a second or subsequent sample cell(s) 22, whereas other embodiments may employ only a single sample cell. Some embodiments may include a spectroscope, spectrometer, spectrum analyzer, dispersive diode array spectrometer, tunable wavelength source or device or tunable laser 24.

Apparatus 10 is operative to measure hydrocarbon contamination in water, e.g., oil and/or grease contamination in water, by passing light through an experimental sample contained within sample cell 14 (e.g., the experimental sample being a water sample that is contaminated or potentially contaminated with hydrocarbons, such as trace hydrocarbon amounts or greater), and by analyzing the light transmitted through the sample to determine the light loss at particular wavelengths or reciprocal wavelengths expressed as wavenumbers or frequencies or within a range of frequencies or wavenumbers in the near infrared (NIR) spectrum. In some embodiments, the results of the experimental sample analysis are compared with the results of an infrared analysis of a reference sample, e.g., pure water, in order to determine the presence of, and in some embodiments, the amount of hydrocarbon contamination. For example, the light loss associated with the reference sample at particular wavenumbers may be subtracted from the light loss associated with the experimental sample at the same wavenumbers, thus yielding light loss associated with the impurities in the experimental sample. As discussed herein, the NIR wavenumbers which are analyzed are those associated with hydrocarbon absorption, and hence, light loss associated with those frequencies is reflective of hydrocarbon contamination. In some embodiments, sample cell 14 may be used to sequentially analyze the reference sample and the experimental sample, e.g., by analyzing the reference sample, flushing the reference sample out of the sample cell and replacing it with the experimental sample, and subsequently analyzing the experimental sample. Other embodiments may reverse the order of analysis.

In some embodiments, the experimental sample may be contained in sample cell 14 and the reference sample may be contained in sample cell 22, and the samples may be analyzed separately. For example, some embodiments may employ a single optical path, and may sequentially analyze the samples, e.g., by analyzing a reference sample in sample cell 14, wherein sample cell 14 being disposed within the optical path; replacing sample cell 14 with sample cell 22 containing an experimental sample, so that sample cell 22 is disposed within the optical path; and then analyzing the experimental sample. The order may be reversed in other embodiments. In some embodiments, dual optical paths, each having the same optical characteristics, may be employed, eliminating the need for replacing the sample cells.

Spectral analysis in some embodiments of the present invention includes directing light generated by an infrared source, e.g., light source 12, through a sample and measuring the loss of light by the sample for different wavelengths, e.g., as determined by means of a spectrometer or spectrum analyzer that separates the light into different wavelengths or frequencies, and an infrared detector that converts the light into measurable signals. In some embodiments, the loss of light by the sample is isolated from other variations in light intensity by use of a reference spectrum, e.g., the analysis of the reference sample, such as a pure water sample in the same sample cell or the same type of sample cell, i.e., having the same optical characteristics. For example, as mentioned above, analysis of the reference sample may employ the same optical path, except for the absence of the experimental sample, which in some embodiments is replaced with the reference sample. If impurities in an experimental sample are to be determined, such as oil or other hydrocarbon impurities in water, a pure water sample may be employed as a reference sample.

While it is possible to measure hydrocarbon contamination in water using infrared analysis, previous apparatuses and methods do not allow the direct measurement of very low concentrations, e.g., trace amounts measured in the tens of parts per million (ppm) or less. For example, a 10 micrometer (0.01 mm) film of water is infrared transparent over a wide spectral range and can be analyzed by general infrared transmission spectroscopy. A sample of water-hydrocarbon mixture can be analyzed by infrared analysis when the infrared beam path length through the sample is restricted to 10 micrometers or less. By the Beer-Lambert law the measured absorbance is proportional to the concentration of the absorbing medium and the thickness. The path length being very low (e.g., 10 µm) for water samples, only relatively high concentrations of hydrocarbons can be measured by general infrared transmission spectroscopy. Typical measurable hydrocarbon concentrations in water are >1%. Current infrared spectrometers do not have the extraordinarily high sensitivity and stability that would be required to extend the measurable hydrocarbon concentrations down to trace amounts or ppm levels using existing methodology. Trace amounts or ppm levels of hydrocarbon contamination in water includes contamination in the range of, for example, tens of ppm, i.e., 100 ppm or less, and in some cases as low as 1-20 ppm (0.0001% - 0.0020%) or less. Increasing the infrared beam path length to, for example, 100 micrometers (0.1 mm) may permit the measurement of lower concentrations of hydrocarbons, but, unfortunately, water may no longer be transparent at higher water sample path lengths, which may impair or prevent measurement of trace amounts of hydrocarbon contamination using existing methodologies. For example, with reference to FIG. 2, water absorbance at 10 µm and 100 µm path lengths are illustrated, indicated by reference characters 30 and 32, respectively. Although increasing the path length from 10 µm to 100 µm may yield measurement of 10x lower concentrations of hydrocarbon contamination, the water sample is no longer transparent over a wide spectral range and the infrared analysis capability may be significantly diminished. It will be understood that further increases in the sample path length will further and substantially reduce the spectral regions where the water sample is transparent, and further impair or prevent measurement of lower amounts of contamination using existing methodologies.

Conventionally, the practice of determining ppm level concentrations of hydrocarbons in water includes first extracting the hydrocarbon from a known quantity of water and then carrying out a determination of the quantity of hydrocarbon extracted. Extraction can be done by solvent extraction, or membrane extraction, for example. Using an infrared transparent solvent, such as a chlorofluorocarbon (CFC), permits determination of extracted hydrocarbon by infrared analysis using several cm path length through the CFC sample.

The inventors have determined that a particular region within the NIR spectrum may be particularly useful for infrared analysis of trace levels of hydrocarbon contamination in water, without requiring the time consuming step of extracting the hydrocarbons from the water prior to analysis.

In one form, apparatus 10 is constructed to measure low levels of spectrally resolved absorbance, e.g., such as for example an FT IR spectrometer in conjunction with a suitably bright infrared source and a suitably sensitive infrared detector element or a suitably stable tunable laser or other device capable of providing highly sensitive and stable spectrometric data. By increasing the path length through the water and hydrocarbon sample such that extensive parts of the infrared spectrum are no longer transparent, while retaining a limited spectral region of transparency coincident with a spectral region where the majority of hydrocarbons (oils, fats and greases) have characteristic absorption bands, the measurement of trace hydrocarbon contamination may be performed. For example, referring to FIGS. 2-5, although it may be considered that one of the strongest characteristic spectral absorption bands attributed to many hydrocarbons is the C-H stretch band at approximately 3450 nm (2900 cm-1), in this spectral region the water absorption (FIG. 2) is still very high due to the broad nature of the O-H stretch band of water at 2770 nm (3600 cm⁻¹), and hence this does not provide a favorable spectral region for the trace or ppm level determination of hydrocarbon contamination in water.

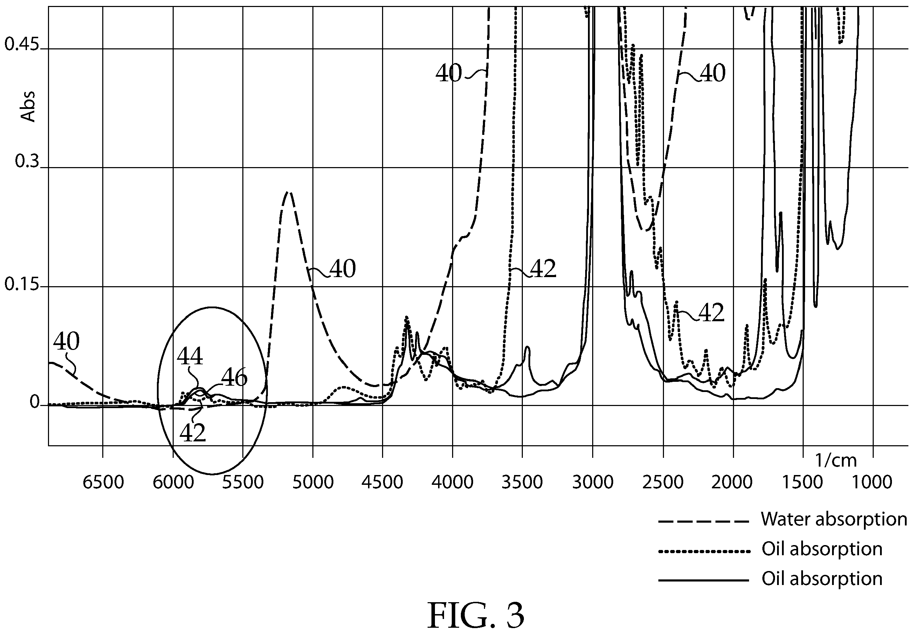

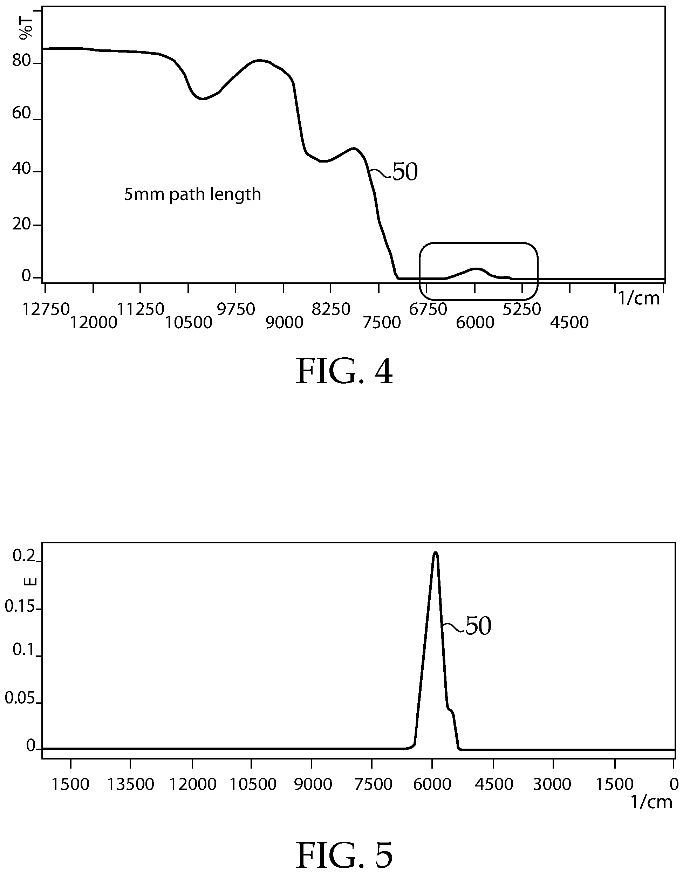

On the other hand the first harmonic of the C-H stretch band at 1770 nm (5700 cm⁻¹) in the near infrared is strong compared with the residual water absorption in this region. The sample path length is maximized by limiting the water transmission to a small but adequate level so that the lowest concentration of hydrocarbons can be measured. FIG. 3 illustrates water absorption 40 and hydrocarbon absorption 42, 44 and 46 for three different hydrocarbons. As illustrated in FIG. 3, the region of approximately 5700 cm⁻¹ to approximately 6300 cm⁻¹, and in particular, the region of approximately 5700 cm⁻¹ to approximately 6000 cm⁻¹, i.e., which includes the C-H stretch first overtone, is a region that includes not only relatively low water absorption, but also includes a region of relatively high hydrocarbon absorption, wherein the ratio of hydrocarbon absorbance to water absorbance is the greatest. On an absolute scale, the hydrocarbon absorption is low in these regions, and thus not a conventionally desirable region for infrared analysis using existing methodologies. However, the inventors have discovered that the relatively high ratio of hydrocarbon absorbance to water absorbance in this region, not previously exploited, allows the measurement of trace levels of hydrocarbon contamination. FIGS. 4 and 5 illustrate transmittance 50 for a 5 mm path length water sample, wherein the water transmittance is approximately 5% in the region of 5700 cm⁻¹. The absorbance of the C-H stretch first overtone (e.g., of hydrocarbons) is much stronger than near infrared bands at shorter wavelengths and hence the high transmittance at frequencies higher than 7000 cm⁻¹ in FIG. 4 is not useful. Some embodiments employ an optical filter, e.g., optical filter 20, such as a 1500 nm long wave filter, which blocks the region above about 7000 cm⁻¹, yielding a narrow region of transmittance near approximately 5700 cm⁻¹ to approximately 6300 cm⁻¹, as illustrated in FIG. 5. In FIGS. 4 and 5, the region below the C-H stretch overtone, e.g., below about 5500 cm⁻¹, is also filtered out for the sake of clarity. The high ratio of hydrocarbon absorbance to water absorbance around the first harmonic for the C-H stretch overtone allows for greater infrared analysis sensitivity to low or trace hydrocarbon contamination quantities, particularly when the path length is increased, e.g., to 5 mm, allowing the measurement of trace amounts of hydrocarbon contamination in the low ppm range, e.g., down to 5 ppm or less in some embodiments, and down to 1 ppm or less in other embodiments.

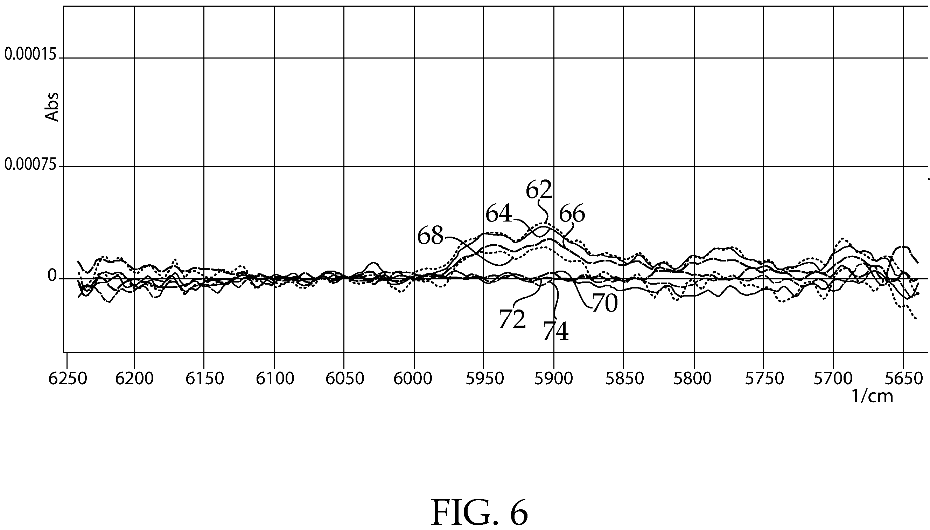

With reference to FIG. 3, although hydrocarbon absorption in the range of about 4200 cm⁻¹ to 4400 cm⁻¹ is greater in an absolute sense than in the region of approximately 5700 cm⁻¹ to approximately 6300 cm⁻¹, the ratio of hydrocarbon absorption to water absorption in the range of about 4200 cm⁻¹ to 4400 cm⁻¹ is not nearly as great as the ratio of hydrocarbon absorption to water absorption in the region of approximately 5700 cm⁻¹ to approximately 6300 cm⁻¹, particularly in the region of about 5700 cm⁻¹ to approximately 6000 cm⁻¹, which makes the latter NIR regions particularly suitable for detecting down to trace amounts of hydrocarbon contamination. The total absorption of radiation outside the small region of transmittance near approximately 5700 cm⁻¹ to approximately 6300 cm⁻¹ is favorable for an FTIR spectrometer avoiding overloading and excess noise when employing a sensitive near infrared detector. FIG. 6 illustrates absorbance spectra of four (4) traces 62, 64, 66, 68 of 20 ppm isopropanol in water and three (3) traces 70, 72, 74 of absorbance for pure water with a 5 mm sample path length. The detection limit in the example of FIG. 6 is estimated at < 5 ppm. It will be understood that lower detection limits may be achieved in other embodiments, e.g., depending upon the light source characteristics and the detector characteristics, and spectrometer characteristics for embodiments so equipped.

Accordingly, the present invention is directed to performing infrared analysis of water samples in the spectral range of only approximately 5700 cm⁻¹ to approximately 6300 cm⁻¹, or wavenumber range between approximately 5700 and 6300. In some embodiments, a more narrow region may be employed, e.g., infrared analysis at or only at wavenumbers between approximately 5700 and 6000.

Light source 12 is operative to supply light or radiation to sample cell 14 for infrared analysis of the sample disposed within sample cell 14, including light in the NIR spectrum in between approximately 5700 cm⁻¹ and 6300 cm⁻¹. Light outside this range may also be supplied by light source 12. In one form, light source 12 is a filament-based incandescent light source, e.g., a commercially available quartz halogen light bulb, such as a quartz halogen automotive light bulb. In other embodiments, light source 12 may take other forms, and may be, for example, one or more lasers and/or light emitting diodes (LEDs) or any light source capable of producing infrared light across range between approximately 5700 cm⁻¹ and 6300 cm⁻¹, and in some embodiments, across the range between approximately 5700 cm⁻¹ and 6000 cm⁻¹.

Spectroscope 24 is constructed to modulate the frequency of the light received from light source 12. For example, spectroscope 24 is constructed to separate the light into separate frequencies or wavelengths, and scan the desired frequencies or wavenumbers of light or radiation within a desired range, e.g., sequentially between the range of approximately 5700 cm⁻¹ and 6300 cm⁻¹, and in some embodiments between the range of approximately 5700 cm⁻¹ and 6000 cm⁻¹. In some embodiments, spectroscope 24 is operative to modulate the light or radiation received from light source 12 only through frequencies in the range of approximately 5700 cm⁻¹ and 6300 cm⁻¹, and in other embodiments only through frequencies between the range of approximately 5700 cm⁻¹ and 6000 cm⁻¹. The frequency increments may vary with the needs of the application. For example, in some embodiments, spectroscope 24 may scan the frequency range in 10 cm⁻¹ increments, whereas other embodiments may employ 100 cm⁻¹ increments. In some embodiments, only certain select frequencies within the desired range may be employed. In one form, spectroscope 24 is a dispersive diode array spectrometer or a variable filter spectrometer. In another form, the source 12 may be a tunable diode laser in which case spectroscope 24 is not required. In a particular form, spectroscope 24 is a Fourier transform infrared (FTIR) spectrum analyzer. A non-limiting example of a suitable spectroscope 24 is a commercially available FTIR spectrum analyzer, such as a MB3000 FTIR spectrum analyzer, manufactured by ABB Bomem of Quebec, Canada. In other embodiments, other spectroscopes or spectrum analyzers may be employed.

In one form, spectroscope 24 is disposed in the optical path between light source 12 and the sample cell being analyzed, e.g., sample cell 14, which avoids the spectral analysis of self emission by the sample, such as self emission in the long wavelength infrared region. In other embodiments, spectroscope 24 may be disposed in the optical path between the sample cell and detector 18, e.g., between the sample cell and filter 20 or between filter 20 and detector 16. Some embodiments may not employ a spectroscope. For example, in some embodiments, detector 16 may be constructed and operative to measure discrete frequencies or frequency bands without the need for a spectroscope to modulate or disperse the light or radiation emanating from light source 12. Some embodiments may only measure the loss of light at two discrete wavelengths or two discrete wavelength bands in order to ascertain the presence and quantity of hydrocarbon contaminant in the experimental sample in cell 14 thereby forgoing a detailed spectral analysis which would permit determining the type or chemical nature of hydrocarbon contaminant For example, a plurality of emitters such as discrete NIR lasers and/or LEDs and/or other light sources may be employed, wherein each emitter discharges light or radiation at a discrete frequency or frequency band in the range between approximately 5700 cm⁻¹ and 6300 cm⁻¹, or in some embodiments in the range between approximately 5700 cm⁻¹ and 6000 cm⁻¹.

Sample cell 14 is constructed to admit and hold a desired quantity of water as a sample S to be analyzed, e.g., either pure water (reference sample) or ostensibly contaminated water (experimental sample). During infrared analysis, sample cell 14 is disposed and positioned within the optical path to receive light generated by light source 12, e.g., as resolved or modulated by spectroscope 24. In one form, sample cell 14 is formed of quartz, i.e., has quartz windows 14A. In other embodiments, other infrared-transparent materials may be employed as windows 14A, e.g., materials that are transparent at frequencies in the range between approximately 5700 cm⁻¹ and 6300 cm⁻¹ in some embodiments or in the range between approximately 5700 cm⁻¹ and 6000 cm⁻¹ in other embodiments. In one form, sample cell 14 has an optical path length PL through the water sample of 5 mm or approximately 5 mm. In other embodiments, the path length may vary with the needs of the application, and may be, for example, in the range of 0.5 mm to 20 mm, or more preferably, in the range of 0.5 mm to 10 mm, or even more preferably, in the range of 2 mm to 8 mm. In other embodiments, the path length may be outside of these ranges. Sample cell 22 for embodiments so equipped is similar to sample cell 14.

Detector 16 is a light or electromagnetic radiation detector positioned to receive light or radiation transmitted through the sample and the sample cell. Detector 16 is operative to detect infrared radiation at least in the range between about 5700 cm⁻¹ and 6300 cm⁻¹ in some embodiments, and at least in the range between about 5700 cm⁻¹ and 6000 cm⁻¹ in other embodiments. Detector 16 may also detect light or radiation at other frequencies. Detector 16 is operative to generate an electronic signal in response to detecting the light or radiation, wherein the signal is indicative of the amplitude or strength of the light or radiation that is received by detector 16. An example of a suitable detector is a commercially available TE cooled extended wavelength InGaAs detector with a wavelength cutoff of at least 2000 nm. In some embodiments, detector 16 may be configured to resolve the frequencies of the IR radiation it detects.

Controller 18 is communicatively coupled to detector 16 and to spectroscope 24. Controller 18 is operative to control the modulation of the light by spectroscope 24. In some embodiments, controller 18 is also communicatively coupled to light source 12 and operative to control the output of light source 12. Controller 18 is operative to receive the signals output by detector 16, and to determine light loss through the sample cell in the range between about 5700 cm⁻¹ and 6300 cm⁻¹ using the signals. In one form, the light loss is denoted measured in terms of absorbance, e.g., such as the absorbance spectra of FIG. 6. In other embodiments, light loss may take other forms, including, for example, transmittance. In some embodiments, the light loss determination may be based on an initial calibration or reference value establishing initial characteristics, e.g., amplitudes at desired frequencies, of the light prior to introduction of the sample cell or of the sample into the optical path. In some embodiments, controller 18 is operative to determine light loss only in the range between about 5700 cm⁻¹ and 6300 cm⁻¹. In other embodiments, controller 18 may be operative to determine light loss only in the range between about 5700 cm⁻¹ and 6000 cm⁻¹. In still other embodiments, controller 18 may be operative to determine light loss at other frequencies as well.

Controller 18 is operative to determine a level of hydrocarbon contamination in the experimental water sample based on the light loss. In some embodiments, controller 18 is operative to compare light loss through the sample cell when filled with a reference water sample, e.g., pure water, to light loss through the sample cell when filled with an experimental water sample, i.e., the ostensibly contaminated water, to determine a light loss difference, and to determine the level of hydrocarbon contamination based on the difference. According to the invention, controller 18 is operative to determine the level of hydrocarbon contamination based on only the light loss in the range between about 5700 cm⁻¹ and 6300 cm⁻¹, or based on only the light loss in the range between about 5700 cm⁻¹ and 6000 cm⁻¹. Controller 18 is operative to generate an output indicating the level of hydrocarbon contamination in the experimental water sample. The output may be, for example, displayed on a display (not shown) and/or may be a printed output.

Filter 20 is an optical filter. In one form, filter 20 is disposed in the optical path between the sample cell and detector 16. In one form, filter 20 is operative to block transmittance at wavenumbers above about 7000, i.e., frequencies above about 7000 cm⁻¹. In one form, filter 20 is a 1500 nm long wave filter. In other embodiments, other filters may be used in addition to or in place of a 1500 nm long wave filter. In some embodiments, a filter may be employed to also or alternatively block wavenumbers below the desired range, e.g., below about 5000-5500 cm⁻¹, which may in some embodiments be considered a part of filter 20.

A non-limiting example of methodology for performing infrared analysis for measuring hydrocarbon contamination in water includes directing light from light source 12 to and through the sample cell, for example, sample cell 14 containing the experimental water sample. The light includes near infrared radiation in a range between about 5700 cm⁻¹ and 6300 cm⁻¹. In some embodiments, the light includes near infrared radiation in a range between about 5700 cm⁻¹ and 6000 cm⁻¹. The light transmitted from or through the water sample is detected by detector 16. The light loss through the experimental water sample is then determined, i.e., the light loss in the range between about 5700 cm⁻¹ and 6300 cm⁻¹. In some embodiments, the light loss in the range between about 5700 cm⁻¹ and 6000 cm⁻¹ through the experimental water sample may be determined. In some embodiments, only the light loss in the range between about 5700 cm⁻¹ and 6300 cm⁻¹ or only the light loss in the range between about 5700 cm⁻¹ and 6000 cm⁻¹ may be determined.

The level of hydrocarbon contamination in the experimental water sample is then determined based only on the light loss in the range between about 5700 cm⁻¹ and 6300 cm⁻¹, or in embodiments, based only on the light loss in the range between about 5700 cm⁻¹ and 6000 cm⁻¹. An output indicating the level of hydrocarbon contamination is then generated, which may include, for example, displaying the level of contamination on a display (not shown) and/or printing the level of contamination using a printing device (not shown).

In some embodiments, the determination of the level of hydrocarbon contamination in the experimental sample is based not only on the light loss through the experimental sample, but also, the light loss through a reference sample, e.g., pure water. For example, light from the light source 12 may be directed through the reference water sample, e.g., pure water; and the light exiting the reference water sample may be detected. The light loss through the reference water sample in a range between about 5700 cm⁻¹ and 6300 cm⁻¹, or in some embodiments in a range between about 5700 cm⁻¹ and 6000 cm⁻¹ is then determined. The light loss through the reference water sample is subtracted from the light loss through the experimental water sample to generate a light loss difference. The level of hydrocarbon contamination in the experimental water sample is then determined based only on the light loss difference in the range between about 5700 cm⁻¹ and 6300 cm⁻¹, or in embodiments, based only on the light loss difference in the range between about 5700 cm⁻¹ and 6000 cm⁻¹. In still other embodiments, the level of hydrocarbon contamination is determined based on the light loss at only one or two discrete wavelengths or wavelength bands selected in the range between 5700 cm⁻¹ and 6300 cm⁻¹.

Various embodiments may include modulating the light prior to reaching the experimental sample (and the reference sample, for embodiments that employ a reference sample) or in some embodiments, the light after being transmitted from the experimental sample (and the reference sample, for embodiments that employ a reference sample). The modulation includes modulating or scanning through desired light frequencies in the range between about 5700 cm⁻¹ and 6300 cm⁻¹ or the range between about 5700 cm⁻¹ and 6000 cm⁻¹ in some embodiments, e.g., sequentially exposing the sample to different frequencies within the desired range. For example, the light may be modulated by a Fourier transform IR spectrum analyzer, e.g., as described above. In other embodiments, light source 12 may modulate the light with modulation frequencies unique to different wavelengths directed to the sample. In still other embodiments, the light may not be modulated - rather, detector 16 may be constructed and operative to resolve the amplitudes of frequency components transmitted through the sample and received at the detector. In some embodiments, the light or radiation is filtered prior to reaching detector 16 using filter 20, e.g., a 1500 nm long pass filter that is operative to block transmittance at wavenumbers above about 7000 cm⁻¹. In other embodiments, other filter parameters may be employed. Some embodiments may also filter out light or radiation below about 5000-5500 cm⁻¹. In other embodiments, other filter parameters may be employed.

In some embodiments, the level of contamination of the experimental sample is also determined by comparing the measured or determined light loss (or light loss difference, in some embodiments), with known light loss (e.g., known light loss values or profiles or characteristics) within the desired range (e.g., the range between about 5700 cm⁻¹ and 6300 cm⁻¹ or the range between about 5700 cm⁻¹ and 6000 cm⁻¹ in some embodiments). The determination of the level of contamination of the experimental sample is then based on the comparison. For example, the known light loss values or profiles may be obtained by measuring samples having known hydrocarbon contamination levels to obtain corresponding light loss characteristics in the range between about 5700 cm⁻¹ and 6300 cm⁻¹ (or the range between about 5700 cm⁻¹ and 6300 cm⁻¹, in some embodiments), yielding light loss characteristics associated with known hydrocarbon contamination levels or known light loss characteristics. The light loss characteristics (or light loss difference characteristics) associated with experimental samples may then be compared to the known light loss characteristics to determine the contamination level of the experimental sample, e.g., by comparison, and in some embodiments, with interpolation. An example of known light loss characteristics is illustrated in FIG. 6 and absorbance spectra of four (4) traces 62, 64, 66, 68 of 20 ppm isopropanol in water and three (3) traces 70, 72, 74 of absorbance for pure water, with a 5 mm sample path length.

The determination of the level of hydrocarbon contamination is based only on light loss (or light loss difference, in some embodiments) in the range between about 5700 cm⁻¹ and 6300 cm⁻¹ (or only in the range between about 5700 cm⁻¹ and 6000 cm⁻¹, in embodiments).

While the invention has been illustrated and described in detail in the drawings and foregoing description, the same is to be considered as illustrative and not restrictive in character, it being understood that only the preferred embodiments have been shown and described. It should be understood that while the use of words such as preferable, preferably, preferred or more preferred utilized in the description above indicate that the feature so described may be more desirable, it nonetheless may not be necessary and embodiments lacking the same may be contemplated as within the scope of the invention, the scope being defined by the claims that follow. In reading the claims, it is intended that when words such as "a," "an," "at least one," or "at least one portion" are used there is no intention to limit the claim to only one item unless specifically stated to the contrary in the claim. When the language "at least a portion" and/or "a portion" is used the item can include a portion and/or the entire item unless specifically stated to the contrary.

Unless specified or limited otherwise, the terms "mounted," "connected," "supported," and "coupled" and variations thereof are used broadly and encompass both direct and indirect mountings, connections, supports, and couplings. Further, "connected" and "coupled" are not restricted to physical or mechanical connections or couplings.

## Claims

1. A method for performing infrared analysis for measuring hydrocarbon contamination in water, comprising:
providing light from a light source (12), the light including a near infrared-, NIR-, radiation output in a range between about 5700 cm⁻¹ and 6300 cm⁻¹;
directing light from the light source (12) through an experimental water sample;
detecting the light transmitted from the experimental water sample;
determining the light loss through the experimental water sample in the range between about 5700 cm⁻¹ and 6300 cm⁻¹;
determining a level of hydrocarbon contamination in the experimental water sample based on the light loss only in the range between about 5700 cm⁻¹ and 6300 cm⁻¹; and
generating an output indicating the level of hydrocarbon contamination in the experimental water sample.

2. The method of claim 1, further comprising:
comparing the light loss with a known light loss in the range between about 5700 cm⁻¹ and 6300 cm⁻¹ associated with a known level of hydrocarbon contamination in water; and
determining a level of hydrocarbon contamination based on the light loss and the known light loss.

3. The method of claim 1, further comprising selecting the light source (12) to have only one or two wavelengths or wavelength bands prior to the light reaching the experimental sample or after being transmitted from the experimental sample, wherein the wavelengths or wavelength bands occur in the range between about 5700 cm⁻¹ and 6300 cm⁻¹.

4. The method of claim 1, wherein the light source (12) is a tunable laser or a plurality of lasers having different variable wavelengths, wherein the tunable laser permits tuning through at least some frequencies in the range between about 5700 cm⁻¹ and 6300 cm⁻¹, or wherein the plurality of lasers output light at at least some frequencies in the range between about 5700 cm⁻¹ and 6300 cm⁻¹.

5. The method of claim 1, wherein:
- the infrared analysis is performed with a dispersive diode array spectrometer; or
- the infrared analysis is performed with a dispersive scanning spectrometer; or
- the infrared analysis is performed using a Fourier transform infrared spectrum analyzer.

6. The method of claim 1, further comprising:
directing light from the light source (12) through a reference water sample;
detecting the light exiting the reference water sample;
determining the light loss through the reference water sample in a range between about 5700 cm⁻¹ and 6300 cm⁻¹;
subtracting the light loss through the reference water sample from the light loss through the experimental water sample to generate a light loss difference; and
determining the level of hydrocarbon contamination in the experimental water sample based on the light loss difference only in the range between about 5700 cm⁻¹ and 6300 cm⁻¹.

7. The method of claim 1,
- wherein the experimental water sample has a path length of between 0.5 and 10 millimeters; or
- further comprising filtering the light with a long wave filter (20) operative to block transmittance at wavenumbers above about 7000 cm⁻¹ prior to detecting the light.

8. The method of claim 1, further comprising:
measuring water samples with known hydrocarbon contamination levels to obtain known light loss characteristics in the range between about 5700 cm⁻¹ and 6300 cm⁻¹ associated with the known hydrocarbon contamination levels; and
determining the level of hydrocarbon contamination in the experimental water sample based on the light loss and the known light loss characteristics.

9. An apparatus for performing infrared analysis for measuring hydrocarbon contamination in water, comprising:
a light source (12) configured to provide an output including a near infrared-, NIR-, spectral output in a range between about 5700 cm⁻¹ and 6300 cm⁻¹;
a sample cell (14) constructed to admit water and positioned to receive light from the light source (12), wherein the sample cell (14) has a sample path length equal to or greater than about 0.5 millimeters;
a detector (16) positioned to receive light transmitted through the sample cell (14), wherein the detector (16) is operative to detect radiation at least in the range between about 5700 cm⁻¹ and 6300 cm⁻¹; and
a controller (18) communicatively coupled to the detector (16), wherein the controller (18) is operative to determine light loss through the sample cell (14) in the range between about 5700 cm⁻¹ and 6300 cm⁻¹, to determine a level of hydrocarbon contamination in the experimental water sample based on the light loss only in the range between about 5700 cm⁻¹ and 6300 cm⁻¹, and to generate an output indicating the level of hydrocarbon contamination in the experimental water sample.

10. The apparatus of claim 9,
- wherein the light source (12) is constructed to yield only one or two wavelengths or wavelength bands prior to the light reaching the experimental sample or after being transmitted from the experimental sample; and wherein the wavelengths or wavelength bands occur in the range between about 5700 cm⁻¹ and 6300 cm⁻¹; or
- wherein the light source (12) is a tunable laser or a plurality of lasers having different variable wavelengths, wherein the tunable laser permits tuning through at least some frequencies in the range between about 5700 cm⁻¹ and 6300 cm⁻¹, or wherein the plurality of lasers output light at at least some frequencies in the range between about 5700 cm⁻¹ and 6300 cm⁻¹.

11. The apparatus of claim 9,
- wherein the infrared analysis is performed using a dispersive diode array spectrometer; or
- wherein the infrared analysis is performed using a dispersive scanning spectrometer; or
- wherein the infrared analysis is performed using a Fourier transform infrared spectrum analyzer.

12. The apparatus of claim 9,
- wherein the sample path length is between 0.5 and 10 millimeters; or
- wherein the sample path length is 5 millimeters.

13. The apparatus of claim 9, further comprising a light modulator positioned between the light source (12) and the sample cell (14) or between the sample cell (14) and the detector (16), wherein the light modulator is operative to modulate the light through at least some light frequencies in the range between about 5700 cm⁻¹ and 6300 cm⁻¹.

14. The apparatus of claim 9, wherein the controller (18) is operative to determine the level of hydrocarbon contamination in the experimental water sample based only on the light loss at only one or two discrete wavelengths or wavelength bands in the range between about 5700 cm⁻¹ and 6300 cm⁻¹.

15. The apparatus of claim 9, wherein the sample path length is between about 2 millimeters and about 8 millimeters, wherein the apparatus further includes a light modulator positioned between the light source (12) and the sample cell (14) or between the sample cell (14) and the detector (16), and wherein the light modulator is operative to modulate the light through at least some light frequencies in the range between about 5700 cm⁻¹ and 6300 cm⁻¹.

## Patentansprüche

1. Verfahren zum Durchführen einer Infrarotanalyse zum Messen von Kohlenwasserstoffkontamination in Wasser, umfassend:
Liefern von Licht von einer Lichtquelle (12), wobei das Licht eine Nahinfrarot-, NIR-, Strahlung beinhaltet, die in einem Bereich zwischen 5700 cm⁻¹ und 6300 cm⁻¹ ausgegeben wird;
Richten von Licht von der Lichtquelle (12) durch eine experimentelle Wasserprobe;
Detektieren das durch die experimentelle Wasserprobe durchgelassenen Lichts;
Bestimmen des Lichtverlusts durch die experimentelle Wasserprobe in dem Bereich zwischen etwa 5700 cm⁻¹ und 6300 cm⁻¹;
Bestimmen eines Grads von Kohlenwasserstoffkontamination in der experimentellen Wasserprobe auf Basis des Lichtverlusts nur in dem Bereich zwischen etwa 5700 cm⁻¹ und 6300 cm⁻¹; und
Erzeugen einer Ausgabe, die den Grad an Kohlenwasserstoffkontamination in der experimentellen Wasserprobe anzeigt.

2. Verfahren nach Anspruch 1, weiter umfassend:
Vergleichen des Lichtverlusts mit einem bekannten Lichtverlust in dem Bereich zwischen etwa 5700 cm⁻¹ und 6300 cm⁻¹, mit einem bekannten Grad an Kohlenwasserstoffkontamination in Wasser assoziiert; und
Bestimmen eines Grads an Kohlenwasserstoffkontamination auf Basis des Lichtverlusts und des bekannten Lichtverlusts.

3. Verfahren nach Anspruch 1, weiter umfassend das Wählen, dass die Lichtquelle (12) nur eine oder zwei Wellenlängen oder Wellenlängenbänder hat, bevor das Licht die experimentelle Probe erreicht, oder nach dem Durchlassen von der experimentellen Probe, wobei die Wellenlängen oder Wellenlängenbänder in dem Bereich zwischen etwa 5700 cm⁻¹ und 6300 cm⁻¹ auftreten.

4. Verfahren nach Anspruch 1, wobei die Lichtquelle (12) ein abstimmbarer Laser oder mehrere Laser mit unterschiedlichen variablen Wellenlängen ist, wobei der abstimmbare Laser das Abstimmen durch mindestens einige Frequenzen in dem Bereich zwischen etwa 5700 cm⁻¹ und 6300 cm⁻¹ gestattet oder wobei die mehreren Laser Licht mit mindestens einigen Frequenzen in dem Bereich zwischen 5700 cm⁻¹ und 6300 cm⁻¹ ausgeben.

5. Verfahren nach Anspruch 1, wobei:
- die Infrarotanalyse mit einem dispersiven Diodenarrayspektrometer durchgeführt wird; oder
- die Infrarotanalyse mit einem dispersiven Abtastspektrometer durchgeführt wird; oder
- die Infrarotanalyse unter Verwendung eines Fourier-Transformations-Infrarotspektrumsanalysierer durchgeführt wird.

6. Verfahren nach Anspruch 1, weiter umfassend:
Richten von Licht von der Lichtquelle (12) durch eine Referenzwasserprobe;
Detektieren des die Referenzwasserprobe verlassenden Lichts;
Bestimmen des Lichtverlusts durch die Referenzwasserprobe in einem Bereich zwischen etwa 5700 cm⁻¹ und 6300 cm⁻¹;
Subtrahieren des Lichtverlusts durch die Referenzwasserprobe von dem Lichtverlust durch die experimentelle Wasserprobe, um eine Lichtverlustdifferenz zu erzeugen; und
Bestimmen des Grads an Kohlenwasserstoffkontamination in der experimentellen Wasserprobe auf Basis der Lichtverlustdifferenz nur in dem Bereich zwischen 5700 cm⁻¹ und 6300 cm⁻¹.

7. Verfahren nach Anspruch 1,
- wobei die experimentelle Wasserprobe eine Weglänge zwischen 0,5 und 10 Millimetern aufweist; oder
- weiter umfassend das Licht mit einem Langwellenfilter (20), der operativ ist zum Blockieren der Transmittanz von Wellenzahlen über etwa 7000 cm⁻¹ vor dem Detektieren des Lichts.

8. Verfahren nach Anspruch 1, weiter umfassend:
Messen von Wasserproben mit bekannten Kohlenwasserstoffkontaminationsgraden, um bekannte Lichtverlustcharakteristika in dem Bereich zwischen etwa 5700 cm⁻¹ und 6300 cm⁻¹, mit den bekannten Kohlenwasserstoffkontaminationsgraden assoziiert, zu erhalten; und
Bestimmen des Grads an Kohlenwasserstoffkontamination in der experimentellen Wasserprobe auf Basis des Lichtverlusts und der bekannten Lichtverlustcharakteristika.

9. Vorrichtung zum Durchführen einer Infrarotanalyse zum Messen einer Kohlenwasserstoffkontamination in Wasser, umfassend:
eine Lichtquelle (12), die ausgelegt ist zum Liefern einer Ausgabe einschließlich einer Nahinfrarot-, NIR-, Spektralausgabe in einem Bereich zwischen etwa 5700 cm⁻¹ und 6300 cm⁻¹;
eine Probenzelle (14), die so konstruiert ist, dass sie Wasser einlässt, und positioniert ist, um Licht von der Lichtquelle (12) zu empfangen, wobei die Probenzelle (14) eine Probenweglänge größer oder gleich etwa 0,5 Millimetern aufweist;
einen Detektor (16), der positioniert ist zum Empfangen von durch die Probenzelle (14) durchgelassenen Licht, wobei der Detektor (16) operativ ist zum Detektieren von Strahlung mindestens in dem Bereich zwischen etwa 5700 cm⁻¹ und 6300 cm⁻¹; und
einen Controller (18), der kommunikativ an den Detektor (16) gekoppelt ist, wobei der Controller (18) operativ ist zum Bestimmen eines Lichtverlusts durch die Probenzelle (14) in dem Bereich zwischen etwa 5700 cm⁻¹ und 6300 cm⁻¹, zum Bestimmen eines Grads an Kohlenwasserstoffkontamination in der experimentellen Wasserprobe auf Basis des Lichtverlusts nur in dem Bereich zwischen etwa 5700 cm⁻¹ und 6300 cm⁻¹ und zum Erzeugen einer Ausgabe, die den Grad an Kohlenwasserstoffkontamination in der experimentellen Wasserprobe anzeigt.

10. Vorrichtung nach Anspruch 9,
- wobei die Lichtquelle (12) so konstruiert ist, dass sie nur eine oder zwei Wellenlängen oder Wellenlängenbänder ergibt, bevor das Licht die experimentelle Wasserprobe erreicht oder nachdem es von der experimentellen Probe durchgelassen wird; und wobei die Wellenlängen oder Wellenlängenbänder in dem Bereich zwischen etwa 5700 cm⁻¹ und 6300 cm⁻¹ auftreten; oder
- wobei die Lichtquelle (12) ein abstimmbarer Laser oder mehrere Laser mit unterschiedlichen variablen Wellenlängen ist, wobei der abstimmbare Laser das Abstimmen durch mindestens einige Frequenzen in dem Bereich zwischen etwa 5700 cm⁻¹ und 6300 cm⁻¹ gestattet oder wobei die mehreren Laser Licht mit mindestens einigen Frequenzen in dem Bereich zwischen 5700 cm⁻¹ und 6300 cm⁻¹ ausgeben.

11. Vorrichtung nach Anspruch 9,
- wobei die Infrarotanalyse mit einem dispersiven Diodenarrayspektrometer durchgeführt wird; oder
- wobei die Infrarotanalyse mit einem dispersiven Abtastspektrometer durchgeführt wird; oder
- wobei die Infrarotanalyse unter Verwendung eines Fourier-Transformations-Infrarotspektrumsanalysierers durchgeführt wird.

12. Vorrichtung nach Anspruch 9,
- wobei die Abtastweglänge zwischen 0,5 und 10 Millimetern beträgt; oder
- wobei die Abtastweglänge 5 Millimeter beträgt.

13. Vorrichtung nach Anspruch 9, weiter umfassend einen Lichtmodulator, der zwischen der Lichtquelle (12) und der Probenzelle (14) oder zwischen der Probenzellen (14) und dem Detektor (16) positioniert ist, wobei der Lichtmodulator operativ ist zum Modulieren des Lichts durch mindestens einige Lichtfrequenzen in dem Bereich zwischen etwa 5700 cm⁻¹ und 6300 cm⁻¹.

14. Vorrichtung nach Anspruch 9, wobei der Controller (18) operativ ist zum Bestimmen des Grads an Kohlenwasserstoffkontamination in der experimentellen Wasserprobe nur auf Basis des Lichtverlusts bei nur einer oder zwei diskreten Wellenlängen oder Wellenlängenbändern in dem Bereich zwischen etwa 5700 cm⁻¹ und 6300 cm⁻¹.

15. Vorrichtung nach Anspruch 9, wobei die Probenweglänge zwischen etwa 2 Millimetern und etwa 8 Millimetern liegt, wobei die Vorrichtung weiter einen Lichtmodulator enthält, der zwischen der Lichtquelle (12) und der Probenzelle (14) oder zwischen der Probenzelle (14) und dem Detektor (16) positioniert ist, und wobei der Lichtmodulator operativ ist zum Modulieren des Lichts durch mindestens einige Lichtfrequenzen in dem Bereich zwischen etwa 5700 cm⁻¹ und 6300 cm⁻¹.

## Revendications

1. Procédé destiné à effectuer une analyse infrarouge pour mesurer la contamination aux hydrocarbures dans l'eau, comprenant :
la délivrance de lumière depuis une source de lumière (12), la lumière comportant un rayonnement de sortie dans le proche infrarouge, NIR, dans une gamme comprise entre environ 5700 cm⁻¹ et 6300 cm⁻¹ ;
l'acheminement de lumière provenant de la source de lumière (12) à travers un échantillon d'eau expérimental ;
la détection de la lumière transmise depuis l'échantillon d'eau expérimental :
la détermination de la perte de lumière à travers l'échantillon d'eau expérimental dans la gamme comprise entre environ 5700 cm⁻¹ et 6300 cm⁻¹ ;
la détermination d'un niveau de contamination aux hydrocarbures dans l'échantillon d'eau expérimental sur la base de la perte de lumière uniquement dans la gamme comprise entre environ 5700 cm⁻¹ et 6300 cm⁻¹ ; et
la génération d'une sortie indiquant le niveau de contamination aux hydrocarbures dans l'échantillon d'eau expérimental.

2. Procédé de la revendication 1, comprenant en outre :
la comparaison de la perte de lumière avec une perte de lumière connue dans la gamme comprise entre environ 5700 cm⁻¹ et 6300 cm⁻¹ associée à un niveau connu de contamination aux hydrocarbures dans l'eau ; et
la détermination d'un niveau de contamination aux hydrocarbures sur la base de la perte de lumière et de la perte de lumière connue.

3. Procédé de la revendication 1, comprenant en outre la sélection de la source de lumière (12) pour avoir uniquement une ou deux longueurs d'onde ou bandes de longueurs d'onde avant que la lumière atteigne l'échantillon expérimental ou après qu'elle a été transmise depuis l'échantillon expérimental, les longueurs d'onde ou bandes de longueurs d'onde se situant dans la gamme comprise entre environ 5700 cm⁻¹ et 6300 cm⁻¹.

4. Procédé de la revendication 1, dans lequel la source de lumière (12) est un laser accordable ou une pluralité de lasers ayant différentes longueurs d'onde variables, dans lequel le laser accordable permet un accord à travers au moins certaines fréquences dans la gamme comprise entre environ 5700 cm⁻¹ et 6300 cm⁻¹, ou dans lequel la pluralité de lasers délivrent de la lumière à au moins certaines fréquences dans la gamme comprise entre environ 5700 cm⁻¹ et 6300 cm⁻¹.

5. Procédé de la revendication 1, dans lequel :
- l'analyse infrarouge est effectuée avec un spectromètre à réseau de diodes dispersif ; ou
- l'analyse infrarouge est effectuée avec un spectromètre à balayage dispersif ; ou
- l'analyse infrarouge est effectuée au moyen d'un analyseur de spectres infrarouges à transformée de Fourier.

6. Procédé de la revendication 1, comprenant en outre :
l'acheminement de lumière provenant de la source de lumière (12) à travers un échantillon d'eau de référence ;
la détection de la lumière sortant de l'échantillon d'eau de référence ;
la détermination de la perte de lumière à travers l'échantillon d'eau de référence dans une gamme comprise entre environ 5700 cm⁻¹ et 6300 cm⁻¹ ;
la soustraction de la perte de lumière à travers l'échantillon d'eau de référence de la perte de lumière à travers l'échantillon d'eau expérimental pour générer une différence de perte de lumière ; et
la détermination du niveau de contamination aux hydrocarbures dans l'échantillon d'eau expérimental sur la base de la différence de perte de lumière uniquement dans la gamme comprise entre environ 5700 cm⁻¹ et 6300 cm⁻¹.

7. Procédé de la revendication 1,
- dans lequel l'échantillon d'eau expérimental a une longueur de chemin comprise entre 0,5 et 10 millimètres ; ou
- comprenant en outre le filtrage de la lumière avec un filtre à ondes longues (20) opérationnel pour bloquer la transmittance à des nombres d'onde supérieurs à environ 7000 cm⁻¹ avant la détection de la lumière.

8. Procédé de la revendication 1, comprenant en outre :
la mesure d'échantillons d'eau avec des niveaux de contamination aux hydrocarbures connus pour obtenir des caractéristiques de perte de lumière connues dans la gamme comprise entre environ 5700 cm⁻¹ et 6300 cm⁻¹ associées aux niveaux de contamination aux hydrocarbures connus ; et
la détermination du niveau de contamination aux hydrocarbures dans l'échantillon d'eau expérimental sur la base de la perte de lumière et des caractéristiques de perte de lumière connues.

9. Appareil destiné à effectuer une analyse infrarouge pour mesurer la contamination aux hydrocarbures dans l'eau, comprenant :
une source de lumière (12) configurée pour délivrer une sortie comportant une sortie spectrale dans le proche infrarouge, NIR, dans une gamme comprise entre environ 5700 cm⁻¹ et 6300 cm⁻¹ ;
une cellule à échantillon (14) construite pour laisser entrer de l'eau et positionnée pour recevoir de la lumière provenant de la source de lumière (12), la cellule à échantillon (14) ayant une longueur de chemin d'échantillon égale ou supérieure à environ 0,5 millimètre ;
un détecteur (16) positionné pour recevoir de la lumière transmise à travers la cellule à échantillon (14), le détecteur (16) étant opérationnel pour détecter un rayonnement au moins dans la gamme comprise entre environ 5700 cm⁻¹ et 6300 cm⁻¹ ; et
un contrôleur (18) couplé de façon communicante au détecteur (16), le contrôleur (18) étant opérationnel pour déterminer une perte de lumière à travers la cellule à échantillon (14) dans la gamme comprise entre environ 5700 cm⁻¹ et 6300 cm⁻¹, pour déterminer un niveau de contamination aux hydrocarbures dans l'échantillon d'eau expérimental sur la base de la perte de lumière uniquement dans la gamme comprise entre environ 5700 cm⁻¹ et 6300 cm⁻¹, et pour générer une sortie indiquant le niveau de contamination aux hydrocarbures dans l'échantillon d'eau expérimental.

10. Appareil de la revendication 9,
- dans lequel la source de lumière (12) est construite pour générer uniquement une ou deux longueurs d'onde ou bandes de longueurs d'onde avant que la lumière atteigne l'échantillon expérimental ou après qu'elle a été transmise depuis l'échantillon expérimental ; et dans lequel les longueurs d'onde ou bandes de longueurs d'onde se situent dans la gamme comprise entre environ 5700 cm⁻¹ et 6300 cm⁻¹ ; ou
- dans lequel la source de lumière (12) est un laser accordable ou une pluralité de lasers ayant différentes longueurs d'onde variables, dans lequel le laser accordable permet un accord à travers au moins certaines fréquences dans la gamme comprise entre environ 5700 cm⁻¹ et 6300 cm⁻¹, ou dans lequel la pluralité de lasers délivrent de la lumière à au moins certaines fréquences dans la gamme comprise entre environ 5700 cm⁻¹ et 6300 cm⁻¹.

11. Appareil de la revendication 9,
- dans lequel l'analyse infrarouge est effectuée au moyen d'un spectromètre à réseau de diodes dispersif ; ou
- dans lequel l'analyse infrarouge est effectuée au moyen d'un spectromètre à balayage dispersif ; ou
- dans lequel l'analyse infrarouge est effectuée au moyen d'un analyseur de spectres infrarouges à transformée de Fourier.

12. Appareil de la revendication 9,
- dans lequel la longueur de chemin d'échantillon se situe entre 0,5 et 10 millimètres ; ou
- dans lequel la longueur de chemin d'échantillon fait 5 millimètres.

13. Appareil de la revendication 9, comprenant en outre un modulateur de lumière positionné entre la source de lumière (12) et la cellule à échantillon (14) ou entre la cellule à échantillon (14) et le détecteur (16), le modulateur de lumière étant opérationnel pour moduler la lumière à travers au moins certaines fréquences lumineuses dans la gamme comprise entre environ 5700 cm⁻¹ et 6300 cm⁻¹.

14. Appareil de la revendication 9, dans lequel le contrôleur (18) est opérationnel pour déterminer le niveau de contamination aux hydrocarbures dans l'échantillon d'eau expérimental uniquement sur la base de la perte de lumière uniquement à une ou deux longueurs d'onde discrètes ou dans une ou deux bandes de longueurs d'onde dans la gamme comprise entre environ 5700 cm⁻¹ et 6300 cm⁻¹.

15. Appareil de la revendication 9, dans lequel la longueur de chemin d'échantillon se situe entre environ 2 millimètres et environ 8 millimètres, l'appareil comportant en outre un modulateur de lumière positionné entre la source de lumière (12) et la cellule à échantillon (14) ou entre la cellule à échantillon (14) et le détecteur (16), et dans lequel le modulateur de lumière est opérationnel pour moduler la lumière à travers au moins certaines fréquences lumineuses dans la gamme comprise entre environ 5700 cm⁻¹ et 6300 cm⁻¹.
